# EUROPÄISCHE PATENTSCHRIFT

(11) **EP 1 363 731 B1**
(45) Veröffentlichungstag und Bekanntmachung des Hinweises auf die Patenterteilung: **12.10.2005**
(21) Anmeldenummer: 02708317.9
(22) Anmeldetag: 28.01.2002
(51) Int. Cl.: B01J 13/08, B01J 13/02, C09B 67/00, A61K 7/00

(54) **VERFAHREN ZUR HERSTELLUNG BLAUER MIKROKAPSELN**
METHOD FOR PRODUCING BLUE MICROCAPSULES
PROCEDE DE PREPARATION DE MICROCAPSULES BLEUES

(30) Priorität: 09.02.2001 DE 10106446
(43) Veröffentlichungstag der Anmeldung: 26.11.2003
(73) Patentinhaber: Symrise GmbH & Co. KG, 37603 Holzminden (DE)
(72) Erfinder: LAGES, Rita, 37619 Bodenwerder (DE); AICKELE, Frank, 37603 Holzminden (DE); LOGES, Hubert, 37671 Höxter (DE); MACHINEK, Arnold, 37603 Holzminden (DE)
(74) Vertreter: Eisenführ, Speiser & Partner
(86) Internationale Anmeldenummer: PCT/EP2002/000855
(87) Internationale Veröffentlichungsnummer: WO 2002/064246

(56) Entgegenhaltungen:
- GB-A- 1 381 444
- US-A- 3 864 275
- US-A- 3 893 933
- US-A- 4 459 277

## Beschreibung

Die Erfindung betrifft ein Verfahren zur Herstellung von einheitlich gefärbten blauen Mikrokapseln unter Verwendung öllöslicher Farbstoffe. Diese Kapseln finden bevorzugt in der kosmetischen Industrie und der Mundhygiene Anwendung.

Die vorliegende Erfindung beschreibt die Verkapselung einer Mischung aus einem öllöslichen Material und einem Farbstoff, wobei die erhaltenen Kapseln eine blaue bis dunkelblaue Farbe aufweisen. Bevorzugte Verkapselungsmethode ist hier die Koazervation, wobei bevorzugt Gelatine und Gummi Arabicum als umhüllendes Material verwendet werden. Bevorzugt werden die so erhaltenen Kapseln, die eine farblose, transparente Hülle und einen blauen Kern aufweisen, in Produkte auf dem Gebiet der Mundhygiene eingearbeitet.

Verfahren zur Herstellung von Mikrokapseln sind aus der Literatur bekannt, wie beispielsweise beschrieben in US-A-3,341,466.

Aus GB-A- 1 381 444 ist die Lehre bekannt, dass Mikrokapseln mit transparenter Hülle, enthaltend ein Öl und einen Farbstoff bzw. eine Farbstoffsuspension, in Zahnpasten eingearbeitet werden können und diesen dann ein stark gesprenkeltes oder geflecktes bzw. gepunktetes Erscheinungsbild geben. Hierbei dient das ölhaltige Material hauptsächlich als Lösungsmittel bzw. Vehikel für den Farbstoff.

In EP-A- 711 544 ist eine Zahnpasta beschrieben, die Kapseln verwendet, enthaltend Agar als Hauptbestandteil des Umhüllungsmaterial. Die Kapseln haben hierbei eine durchschnittliche Größe von 0,3 bis 3 mm.

Eine spezielle Form der Verkapselung für Mundhygieneprodukte ist beschrieben in WO 99/59535, wobei dort die Aspekte der Stabilität der Kapseln in Gegenwart obenflächenaktiver Substanzen und der Schaumbildung im Vordergrund stehen.

WO 00/48560 befasst sich mit der Verkapselung einer Mischung eines flüssigen Öls und eines Kupfer-Chlorophyll-Extraktes. Bevorzugt werden diese grünen Mikrokapseln in Mundhygieneprodukten eingesetzt. Typische Öle sind Sonnenblumenöl oder Paraffinöl.

Üblicherweise werden die oben erwähnten Mikrokapseln mittels Koazervation hergestellt und der Öl-Kern gegebenenfalls eingefärbt. Zur Herstellung blauer Kapseln gibt es grundsätzlich zwei Möglichkeiten.

Wenn die Hülle der Kapseln mit einem wasserlöslichen Farbstoff vorgenommen wird, tritt Entfärbung der Kapsel ein durch sogenanntes Ausbluten der Farbe oder durch Instabilität der Farbe in der Zubereitung bzw. Formulierung des Anwendungsproduktes.

Wird der öllösliche, d.h. lipophile Kern der Kapsel gefärbt, kommen für diese Art der Einfärbung öllösliche Farbstoffe oder Pigmentfarbstoffe in Betracht. Bei der Verwendung von Pigmentfarbstoffen tritt sehr häufig das Problem der Sedimentation auf, d.h. dass der Farbstoff nicht homogen in der Kapsel verteilt ist und optisch der Eindruck entsteht, die Kapseln sei nur zur Hälfte gefüllt.

Eine zufriedenstellende Einfärbung von Kapseln mit dem Farbton "blau" mit in der Kosmetik zugelassenen Farbstoffen ist bisher nicht gelungen, weil es nur wenige für die Kosmetik zugelassene blaue, öllösliche Farbstoffe gibt.

Derzeit gibt es für die Herstellung blauer Kapseln im kosmetischen Bereich keine Möglichkeit zur Überwindung der beschriebenen Probleme. In den Farbstoff-Listen gibt es keinen öllöslichen Farbstoff, der direkt als "blau" beschrieben wird.

Es bestand daher die Aufgabe, Mikrokapseln mit lipophilen Kern (Kernmaterial), blauer Färbung des Kerns und transparenter Hülle herzustellen, wobei der verwendete Farbstoff ein in der Kosmetik zugelassener, öllöslicher Farbstoff ist.

Es wurden nun zwei öllösliche Farbstoffe gefunden, mit denen eine blaue Färbung des lipophilen Kerns der Kapsel gelingt, wobei die fertiggestellte Mikrokapsel die notwendige Farbstabilität aufweist und weder eine Sedimentation noch ein Ausbluten der Farbe zeigt.

Gegenstand der Erfindung ist daher ein Verfahren zur Herstellung von blauen Mikrokapseln durch Verkapselung einer Mischung eines lipophilen Kernmaterials und eines öllöslichen Farbstoffes, dadurch gekennzeichnet, dass als Farbstoff D&C Grün Nr. 6 oder Guaiazulen oder ein Mischung dieser beiden Farbstoffe eingesetzt wird.

Zum einen hat sich Guaiazulen (7-Isopropyl-1,4-dimethylazulen) bewährt, zum anderen D&C Grün No. 6 (1,4-Bis(4-methylphenyl)-arnino)-9,10-anthracendion; 1,4-p-Toluidinoanthrachinon; C.I. 61565). Letzterer ist besonders überraschend, da dieser in den Farbstoff-Listen als grüner öllöslicher Farbstoff geführt wird.
Beide Substanzen sind kommerziell erhältliche Farbstoffe.

Die Farbstoffe werden, je nach gewünschter Intensität und Tiefe der Blaufärbung, in 0,02 % bis 2 %, bevorzugt in 0,05 % bis 1,5 %, besonders bevorzugt in 0,1 % bis 1 % und ganz besonders bevorzugt in 0,1 % bis 0,5 % dem Kernmaterial zugesetzt. Die Angaben sind Gewichtsprozente und beziehen sich auf die Menge eingesetzten Kernmaterials.

Als Verkapselungsmaterialien, die typischerweise verwendet werden, seien beispielsweise genannt:
Cyclodextrine, Gummi Arabicum, Gelatine, Casein, Albumin, Fibrinogen, Xanthan Gum, lösliche Peptide, Natriumalginat, Carboxymethylcellulose, Polyvinylpyrrolidone und andere natürliche oder synthetische polymere Materialien.

Bevorzugt werden die Verkapselungsmaterialen zusätzlich vemetzt. Die Vernetzung kann dem Verkapselungsmaterial inhärent sein oder durch Zugabe von sogenannten Vernetzern, wie beispielsweise Glutaraldehyd, herbeigeführt werden.

Die Verkapselung kann nach an sich bekannten und in der Literatur beschriebenen Methoden vorgenommen werden. Bevorzugt ist im erfindungsgemäßen Verfahren die Koazervation.

In dem erfindungsgemäßen Verfahren werden bevorzugt Mischungen aus Gummi Arabicum und Gelatine eingesetzt, besonders bevorzugt ist hier ein Mischungsverhältnis von 50 % : 50 % nach Gewichtsprozenten. Vorteilhaft ist die abschließende Vernetzung mit einem Vemetzer, bevorzugt Glutardialdehyd, um die gewünschte Härte der Kapseln zu erreichen.

### Der Herstellprozess kann beispielsweise wie folgt durchgeführt werden:

Das zumeist flüssige Kernmaterial wird mit dem Farbstoff vermischt. Dieses Gemisch, wird danach in eine wässrige Lösung des Hüllenmaterials, bevorzugt enthaltend Gelatine, bei 40-60° eingebracht. Danach kann gegebenenfalls ein weiteres Hüllenmaterial zugegeben werden, bevorzugt Gummi Arabicum. Die wässrige Lösung kann im Bedarfsfalle mit einem Stabilisator oder Konservierungsmittel versetzt werden, bevorzugt ist Kaliumsorbat. Diese Emulsion wird auf einen pH-Wert im Bereich 3.8 bis 4.8 eingestellt, bevorzugt ist ein pH-Bereich von 4.0 bis 4.5. Hierbei bilden sich die Koazervate, d.h. das farbstoffhaltige flüssige Kernmaterial wird von einem Film aus Hüllenmaterial und Wasser umgeben, wobei sich die Mikrokapseln bilden. Nach Abkühlung, zumeist auf Temperaturen unterhalb von 15°C, kann zur Härtung der Schale ein Vernetzer, bevorzugt Glutardialdehyd, zugegeben werden.

Die Reihenfolge der Verarbeitung kann derart abgewandelt werden, dass das farbstoffhaltige Kernmaterial in eine wässrige Lösung aus Gelatine und Gummi Arabicum zugegeben wird und zuletzt die Anpassung des pH-Wertes erfolgt.

Wird bei der Koazervation Fischgelatine verwendet, so liegen die Verarbeitungstemperaturen niedriger, üblicherweise bei 30 bis 45°C.

Nach dem Herstellungsprozess liegen die Kapseln in Form einer Aufschlämmung vor, die als solche weiter eingesetzt werden kann. Es können, je nach Bedarf und Verwendung, weitere Behandlungen folgen. Es können der Aufschlämmung Verdickungsmittel, bevorzugt Carboxymethylcellulose, Konservierungsmittel und Stabilisatoren, beispielsweise Kaliumsorbat und Citrate, zugesetzt werden. Ebenfalls kann die Aufschlämmung bei Bedarf einer Trocknung unterworfen werden, wonach die Mikrokapseln in fließfähiger Form vorliegen.

Die Mikrokapseln weisen eine durchsichtige oder durchscheinende Kapselhülle auf, und enthalten eine blaue Flüssigkeit bzw. ein blaues niedrigschmelzendes Kernmaterial. Somit sind die erfindungsgemäß hergestellten Kapseln gut sichtbar in dem Anwendungsprodukt, beispielsweise einem Mundhygieneprodukt.

Mittels sorgfältiger Anpassung der Bedingungen des Herstellverfahrens können Eigenschaften wie beispielsweise Härte, Größe, Wanddicke, Farbtiefe oder Stabilität der Kapseln maßgeblich beeinflusst werden.

Werden die erfindungsgemäßen blauen Kapseln beispielsweise in Zahnpasten eingearbeitet, ist die Härte der Kapseln durch das erfindungsgemäße Verfahren derart einstellbar, dass die Kapseln weder zu hart sind, um den Putzvorgang zu überdauern, noch zu weich, um während des Herstellprozesses des Mundhygieneproduktes zerstört zu werden. Vielmehr ist es in solch einem Fall Ziel, dass die Kapseln über den gesamten Zeitraum des Putzvorgangs nach und nach zerstört werden und so eine kontrollierte Freisetzung des in den Kapseln enthaltenen lipophilen Materials stattfinden kann.

Typischerweise liegt die Partikelgröße der durch das erfindungsgemäße Verfahren hergestellten blauen Kapseln im Bereich von 0,3 bis 2,5 mm, bevorzugt im Bereich von 0,5 bis 1,8 mm und besonders bevorzugt im Bereich von 0,8 bis 1,2 mm. Die Partikelgröße kann mit üblichen Methoden gemessen werden, wie beispielsweise durch Siebung oder Mikroskopie.

Das erfindungsgemäß zu verkapselnde öllösliche Kernmaterial kann aus einem Einzelstoff oder einer Stoffmischung bestehen. Als geeignete Kemmaterialien seien im folgenden einige beispielhaft genannt.

Neben diversen Speiseölen, Paraffinölen und Silikonölen können die auf dem Gebiet der Kosmetik und der Mundhygiene üblichen Ingredienzien verwendet werden.

Insbesondere seien Materialien genannt, die therapeutische, sensorische, schützende, pflegende oder kosmetische Effekte bewirken.
Proteine, Keratin, Collagen, Casein, Lecithin, Sorbitol, Antioxidantien, Phenolderivate, antimikrobielle Agentien, entzündungshemmende Substanzen, karieshemmende Substanzen, Vitamine, Enzyme, Pflanzenextrakte, Konservierungsmittel, pH-Regulatoren, Süßstoffe, Stärke, Geschmackstoffe und Aromen.

Besonders geeignet als Kernmaterial im Sinne der Erfindung ist die Verwendung von Etherischen Ölen und Extrakten, Tinkturen und Balsame, wie beispielsweise Anisöl, Basilikumöl, Campheröl, Citronellöl, Eucalyptus-citriodora-Öl, Eucalyptusöl, Kamillenöl, Krauseminzöl, Limetteöl, Mandarinenöl, Nelkenöl, Orangenöl, Pfefferminzöl, Salbeiöl, Thymianöl, Vanilleextrakt, Zimtrindenöl, sowie Fraktionen davon, bzw. daraus isolierte Inhaltsstoffe.

Daneben sind besonders als Kernmaterial Substanzen geeignet mit kühlender, erfrischender Wirkung im Mund-, Rachen- oder Nasenraum. Hier seien beispielsweise genannt Menthol, Menthon, Carboxamide, Methonacetale, Mentholcarbonate, Mentholsuccinate, 1,8-Cineol (Eucalyptol), Carvon, alpha-Terpineol, Thymol, Methylsalicylat, 2'-Hydroxypropiophenon. Die optisch aktiven Verbindungen können in enantiomerenreiner Form eingesetzt werden oder als beliebige Gemische der beiden Enantiomere.

Für Anwendungen der erfindungsgemäßen Kapseln in der Kosmetik und Mundhygiene seien genannt: Cremes, Gele, Schäume, Dispersionen, Kaugummis, Lutschpastillen und -bonbons.

Besonders bevorzugt ist der Anwendungsbereich der Zahnpflegeprodukte, insbesondere der der Zahnpasten, Zahncremes, Zahngele.

Die Kapseln werden, je nach zu erzielendem Effekt, der Anwendung im Bereich Mundhygiene in 0,05 % bis 5 %, bevorzugt in 0,2 % bis 3 % und insbesondere bevorzugt in 0,5 % bis 1,5 % zugesetzt. Die Angaben sind Gewichtsprozente und beziehen sich auf die fertige Produktformulierung bzw. die Gesamtrezeptur.

### Die folgenden Beispiele erläutern die Erfindung:

### Beispiel 1:

Es wurde eine Mischung aus Pfefferminzöl und 0,1 % D&C Grün Nr. 6 hergestellt. Diese wurde bei 50°C zu einer wässrigen Gelatine-Lösung zugegeben. Diese Emulsion wurde dann mit einer wässrigen Lösung von Gummi Arabicum versetzt. Der pH-Wert des Gemisches wurde unter Rühren mit wässriger Essigsäure-Lösung auf einen pH-Wert von 4,5 eingestellt. Die Mischung wurde langsam auf Raumtemperatur abgekühlt und anschließend Glutardialdehyd zugegeben. Die überstehende Lösung wurde abdekantiert und die Mikrokapseln mehrmals mit Wasser gewaschen. Die so hergestellten Kapseln hatten einen Durchmesser von 0,6 bis 1,5 mm. Diese Mikrokapseln wiesen eine transparente Hülle und ein blau gefärbtes Kernmaterial auf.

### Beispiel 2:

Es wurde eine Mischung aus Pfefferminzöl und 0,25 % Guaiazulen hergestellt. Diese wurde bei 50°C zu einer wässrigen Gelatine-Lösung zugegeben. Diese Emulsion wurde dann mit einer wässrigen Lösung von Gummi Arabicum versetzt. Der pH-Wert des Gemisches wurde unter Rühren mit wässriger Essigsäure-Lösung auf einen pH-Wert von 4,5 eingestellt. Die Mischung wurde langsam auf Raumtemperatur abgekühlt und anschließend Glutardialdehyd zugegeben. Die überstehende Lösung wurde abdekantiert und die Mikrokapseln mehrmals mit Wasser gewaschen. Die so hergestellten Kapseln hatten einen Durchmesser von 0,5 bis 1,4 mm. Diese Mikrokapseln wiesen eine transparente Hülle und ein blau gefärbtes Kernmaterial auf.

### Beispiel 3:

Es wurde ein transparentes Zahngel mit folgender Grundrezeptur hergestellt, in welches die nach unterschiedlichen Färbemethoden bzw. mit unterschiedlichen Farbstoffen hergestellten blauen Kapseln eingearbeitet wurden.

| Ingredienzien | | Gewichtsteile |
|---|---|---|
| 1. | Sorbitol, 70 %ig | 61,5 |
| 2. | dest. Wasser | 11,4 |
| 3. | Saccharin | 0,2 |
| 4. | Natriummonofluorphosphat | 1,1 |
| 5. | Trinatriumphosphat | 0,1 |
| 6. | Polyethylenglykol PEG 1500 (PEG 32) | 5,5 |
| 7. | Abrasives Kieselgel | 8,0 |
| 8. | Verdickendes Kieselgel | 8,0 |
| 9. | Natriumcarboxymethylcellulose | 0,6 |
| 10. | Natriumlaurylsulfat | 1,5 |
| 11. | Geschmackstoffe | 1,0 |
| 12. | Blaue Kapseln | 1,0 |
| 13. | 4-Hydroxybenzoesäuremethylester | 0,1 |

Bei der Verwendung von mit dem Pigmentfarbstoff Sicomet Blau P 74160 (KupferPhthalocyanin, C.I. 74160; CAS Nr. 147148) gefärbten Kapseln bekam das Zahngel auf Grund der halbmondartigen Färbung der Kapseln ein uneinheitliches Erscheinungsbild, das nicht ansprechend war. Das fertige Zahngel machte den Eindruck einer Fehlcharge und wirkte nicht abgerundet.

### Beispiel 4:

Es wurde bei gleicher Dosierung der nach Beispiel 1 hergestellten Kapseln festgestellt, das das Zahngel den optischen Eindruck einer homogene Verteilung der blauen Kapseln aufwies, die Kapseln fügten sich harmonisch und erfrischend in das Erscheinungsbild ein.

## Patentansprüche

1. Verfahren zur Herstellung von blauen Mikrokapseln durch Verkapselung einer Mischung eines lipophilen Kernmaterials und eines öllöslichen Farbstoffes, **dadurch gekennzeichnet, dass** als Farbstoff D&C Grün Nr. 6 oder Guaiazulen oder ein Mischung dieser beiden Farbstoffe eingesetzt wird.

2. Verfahren nach Anspruch 1, **dadurch gekennzeichnet, dass** die Verkapselung mittels Koazervation durchgeführt wird.

3. Verfahren nach Anspruch 1, **dadurch gekennzeichnet, dass** die Verkapselung mittels Koazervation unter Verwendung von Gelatine und Gummi Arabicum durchgeführt wird.

4. Verfahren nach Anspruch 1, **dadurch gekennzeichnet, dass** die Verkapselungsmaterialien vernetzt werden.

5. Verfahren nach Anspruch 4, **dadurch gekennzeichnet, dass** als Vernetzer Glutardialdehyd verwendet wird.

6. Verwendung der nach dem Verfahren gemäß Anspruch 1 hergestellten Kapseln in kosmetischen Produkten oder Mundhygieneprodukten.

7. Verwendung der nach dem Verfahren gemäß Anspruch 1 hergestellten Kapseln in Zahnpasta, Zahncreme und Zahngel.

## Claims

1. A process for the production of blue microcapsules by encapsulating a mixture of a lipophilic core material and an oil-soluble dye, **characterised in that** the dye used is D&C Green no. 6 or guaiazulene or a mixture of these two dyes.

2. A process according to claim 1, **characterised in that** encapsulation is performed by means of coascervation.

3. A process according to claim 1, **characterised in that** encapsulation is performed by means of coascervation using gelatin and gum arabic.

4. A process according to claim 1, **characterised in that** the encapsulation materials are crosslinked.

5. A process according to claim 4, **characterised in that** glutardialdehyde is used as the crosslinking agent.

6. Use of the capsules produced by the process according to claim 1 in cosmetic products or oral hygiene products.

7. Use of the capsules produced by the process according to claim 1 in tooth paste, tooth cream and tooth gel.

## Revendications

1. Procédé pour la préparation de microcapsules bleues par encapsulation d'un mélange d'un matériau de noyau lipophile et d'un colorant soluble dans l'huile, **caractérisé en ce que** l'on utilise comme colorant du verre D & C n° 6 ou du guaiazulène ou un mélange de ces deux colorants.

2. Procédé selon la revendication 1, **caractérisé en ce que** l'encapsulation est réalisée au moyen d'une coacervation.

3. Procédé selon la revendication 1, **caractérisé en ce que** l'encapsulation est réalisée au moyen d'une coacervation en utilisant de la gélatine et de la gomme arabique.

4. Procédé selon la revendication 1, **caractérisé en ce que** les matériaux d'encapsulation sont réticulés.

5. Procédé selon la revendication 4, **caractérisé en ce que** l'on utilise comme agent de réticulation du glutardialdéhyde.

6. Utilisadon des capsules préparées selon le procédé selon la revendication 1 dans des produits cosmétiques ou dans des produits pour l'hygiène buccale.

7. Utilisation des capsules préparées selon le procédé selon la revendication 1 dans du dentifrice, de la crème dentaire et du gel dentaire.
